# EUROPÄISCHE PATENTANMELDUNG

(11) **EP 3 262 928 A1**
(43) Veröffentlichungstag der Anmeldung: **03.01.2018**
(21) Anmeldenummer: 17172181.4
(22) Anmeldetag: 22.05.2017
(51) Int. Cl.: A01K 1/02, A01K 27/00

(54) **STABILISIERUNGSVORRICHTUNG FÜR TIERE**

(30) Priorität: 30.06.2016 DE 202016004112 U
(71) Anmelder: Thieme, Andreas, 01896 Pulsnitz (DE); Ursinus, Torsten, 01324 Dresden (DE)
(72) Erfinder: Thieme, Andreas, 01896 Pulsnitz (DE); Ursinus, Torsten, 01324 Dresden (DE)
(74) Vertreter: Kailuweit & Uhlemann Patentanwälte Partnerschaft mbB

(57) **Zusammenfassung**

Die Erfindung betrifft eine Stabilisierungsvorrichtung für Tiere zur medizinischen Behandlung, zur Entlastung, Stabilisation, Sicherung sowie dem waagerechten Anheben und Transport, welche verschiedene Bestandteile(1, 2, 3, 4) umfasst. Dazu zählen ein flächig ausgebildetes Bauchelement (1), welches im Bereich des Bauches anordbar ausgebildet ist, und eine flexible und anatomisch anpassbare Auflagefläche (5) und mindestens vier Verbindungselemente (9) mit daran angeordneten ersten Verschlussmitteln (6) aufweist. Weiterhin zählt ein flächig ausgebildetes Rückenelement (2) zu den Bestandteilen der Stabilisierungsvorrichtung, welches im Bereich des Rückens anordbar ausgebildet ist, und zumindest abschnittsweise zu der Längsachse mindestens ein Mittel zur Stabilisierung (10) und zumindest vier zweite korrespondierende Verschlussmittel (7) aufweist. Dabei sind die mindestens vier Verbindungselemente spiegelsymmetrisch zu der Längsachse des Bauchelementes voneinander beabstandet am Bauchelement angeordnet. Weiterhin ist das Bauchelement (1) mit dem Rückenelement (2) durch die Verbindungselemente (9) mit den daran angeordneten ersten Verschlussmitteln (6) und den korrespondierenden zweiten Verschlussmitteln (7) lösbar verbindbar ausgebildet, wobei die Anzahl der ersten Verschlussmittel der Anzahl der korrespondierenden zweiten Verschlussmittel entspricht.

## Beschreibung

Die Erfindung betrifft eine Stabilisierungsvorrichtung für Tiere zur medizinischen Behandlung, zur Entlastung, Stabilisation, Sicherung sowie dem waagerechten Anheben und Transport.
Die Stabilisierungsvorrichtung für Tiere umfasst dabei verschiedene Bestandteile wie ein flächig ausgebildetes Bauchelement mit einer flexiblen und anatomisch anpassbaren Auflagefläche sowie ein flächig ausgebildetes Rückenelement mit zumindest einem Mittel zur Stabilisierung. Die Bestandteile sind miteinander lösbar verbindbar ausgebildet über Verbindungselemente und Verbindungsmittel.

Geschirre, wie beispielsweise Hundegeschirre, dienen dem Halter oder Benutzer zur Führung, Erziehung und Unterstützung des Hundes. In der Regel bestehen sie aus einem Gurt über dem Brust- und Rückenbereich oder nur über den Brust- oder Rückenbereich.

In US 4559906 A ist ein Geschirr für Hunde und Katzen offenbart. Dieses umfasst Gurte, welche jeweils um Bauch und Rücken geschlungen werden sowie einen Vordergurt, welcher um den Hals- bzw. Brustbereich des Tieres verläuft. Durch einen starren Griff, welcher im Rückenbereich an den Gurten angebracht ist, kann das Tier angehoben und getragen werden. Dabei werden die Hubkräfte durch die Ausgestaltung des Griffes gleichmäßig auf den Körper verteilt, was schonend für das Tier ist.

Die US 7325516 B2 beschreibt ein Tragesystem, um schwache oder kranke Tiere speziell beim Treppensteigen zu unterstützen. Das Tragesystem umfasst eine Weste, welche durch Klettverschlüsse um den Tierkörper lösbar befestigt wird. Die Weste bedeckt Brust-, Bauch-, Schulter- und Rückenbereich des Tieres. An der Weste sind verschieden ausgestaltete Griffe und Gurte angebracht. Dabei wird insbesondere Wert auf die Anhebung der Hüfte speziell für kranke oder verletzte Tiere gelegt.

In US 8336506 B2 wird ein Hüft-Hebe-Gurtzeug für Hunde offenbart. Dieses umfasst ein Brust- und Hüftelement, welche jeweils um den Rumpf reichen und miteinander über Gurte verbunden sind. Weiterhin verfügen beide Elemente jeweils über Griffe, an welchen der Hund angehoben werden kann um ihn beispielsweise in ein Fahrzeug zu heben.

Auch in der US 9320260 B2 wird das in der US 8336506 B2 offenbarte Hüft-Hebe-Gurtzeug um Ausführungsbeispiele ergänzt, in denen eine Leine zwischen einem am Brustelement angebrachten D-Ring und dem am Hüftelement angebrachten Griff gespannt ist und als Tragegurt, speziell für größere Tiere, gestaltet ist.

Die aus dem Stand der Technik offenbarten Gurte und Geschirre sind besonders für kleinere Säugetiere wie Hunde und Katzen geeignet.

Weiterhin weisen die offenbarten Gurte und Geschirre erhebliche Mängel hinsichtlich einer anatomisch schonenden Tragweise auf, da sie entweder den Bauch abschnüren, Muskeln und Blutgefäße einklemmen, die Wirbelsäule falsch belasten oder dem Körper des Tieres nicht angemessen anpassbar sind.

Es wäre daher in hohem Maße wünschenswert, ein Geschirr zur Verfügung zu stellen, welches so ausgestaltet ist, dass es dem Tier eine angenehme Fixierung und Lagerung sowohl in ruhender Position als auch beim Transport und Anheben ermöglicht. Das Geschirr soll weiterhin auch für größere Säugetiere geeignet sein.

Aufgabe ist es, ein Geschirr für Tiere bereitzustellen, welches die Nachteile des Standes der Technik überwindet.

Dabei soll der Bewegungsapparat des Tieres bei Verletzungen, nach Operationen oder altersbedingt, geschont werden. Weiterhin soll das Tier durch das Geschirr körperentlastend angehoben und transportiert werden, bei einer gleichzeitig erwünschten stabilen Lagerung und Sicherung im Geschirr, ohne eine Belastung, ein Abschnüren oder Einengen hervorzurufen.

Weiterhin soll dem Halter des Tieres eine komfortable und bequeme Handhabung des Geschirrs beim Anheben und Transport des Tieres oder beim Gassi gehen ermöglicht werden.

Erfindungsgemäß wird die Aufgabe durch eine Stabilisierungsvorrichtung für Tiere gemäß den unabhängigen Ansprüchen gelöst. Vorteilhafte Ausgestaltungen der Erfindung sind in den abhängigen Ansprüchen angegeben.

Ein erster Aspekt der Erfindung betrifft eine Stabilisierungsvorrichtung für Tiere zur medizinischen Behandlung, zur Entlastung, Stabilisation, Sicherung sowie dem waagerechten Anheben und Transport, welche verschiedene Bestandteile umfasst. Dazu zählt ein flächig ausgebildetes Bauchelement, welches im Bereich des Bauches anordbar ausgebildet ist, und eine flexible und anatomisch anpassbare Auflagefläche, welche mindestens vier Verbindungselemente mit daran angeordneten ersten Verschlussmitteln aufweist. Die Stabilisierungsvorrichtung umfasst weiterhin ein flächig ausgebildetes Rückenelement zu den Bestandteilen der Stabilisierungsvorrichtung, welches im Bereich des Rückens anordbar ausgebildet ist, und zumindest abschnittsweise zu der Längsachse mindestens ein Mittel zur Stabilisierung und zumindest vier zweite Verschlussmittel aufweist. Dabei sind die mindestens vier Verbindungselemente spiegelsymmetrisch zu der Längsachse des Bauchelementes voneinander beabstandet am Bauchelement angeordnet Weiterhin ist das Bauchelement mit dem Rückenelement durch die Verbindungselemente mit den daran angeordneten ersten Verschlussmitteln und den korrespondierenden zweiten Verschlussmitteln lösbar verbindbar ausgebildet, wobei die Anzahl der ersten Verschlussmittel der Anzahl der korrespondierenden zweiten Verschlussmittel entspricht.

In einer Ausführungsform handelt es sich um eine Stabilisierungsvorrichtung für Tiere. In einer Ausführungsform wird unter dem Begriff Tier jedes Säugetier verstanden. In einer Ausführungsform wird unter Tier jedes Lebewesen (mit Ausnahme des Menschen) verstanden. In einer weiteren Ausführungsform wird unter dem Begriff Tier jedes Tier mit vier Beinen verstanden. Ein Tier mit vier Beinen wird auch als Vierbeiner bezeichnet. Darunter fallen insbesondere auch Gebrauchs- und Nutztiere. Weiterhin umfasst der Begriff Vierbeiner Hunde, Katzen, Pferde, insbesondere Renn- und Sportpferde und Kamele.

Im Folgenden wird die Stabilisierungsvorrichtung für Tiere verkürzt als Stabilisierungsvorrichtung bezeichnet.

In einer Ausführungsform umfasst die Stabilisierungsvorrichtung verschiedene Bestandteile.

In einer Ausführungsform weist die Stabilisierungsvorrichtung zumindest teilweise reflektierende Elemente auf. Vorteilhaft sind die reflektierenden Elemente an der in die Umgebung zeigende Oberfläche der Stabilisierungsvorrichtung angebracht, sodass der Träger der Stabilisierungsvorrichtung durch die Reflexion im Straßenverkehr auch im Dunkeln oder bei schlechten Lichtverhältnissen gut erkennbar ist.

In einer Ausführungsform umfassen die Bestandteile der Stabilisierungsvorrichtung ein Bauchelement und ein Rückenelement. In einer bevorzugten Ausführungsform umfassen die Bestandteile der Stabilisierungsvorrichtung ein Bauchelement, ein Rückenelement und ein Vorderteil. Vorteilhaft wird damit der komplette Rumpf des Tieres von der Stabilisierungsvorrichtung umgeben.

In einer Ausführungsform ist das Vorderteil als handelsübliches Geschirr ausgebildet.

In einer bevorzugten Ausführungsform weist das Vorderteil ein Brustelement und ein Schulterelement auf. Somit kann die erfindungsgemäße Stabilisierungsvorrichtung mit einem handelsüblichen Gurt oder Geschirr verwendet werden. In einer weiteren Ausführungsform umfassen die Bestandteile der Stabilisierungsvorrichtung ein Bauchelement, ein Rückenelement, ein Brustelement und ein Schulterelement.

In einer Ausführungsform ist die Stabilisierungsvorrichtung vollständig in die einzelnen Bestandteile zerlegbar ausgebildet. In einer weiteren Ausführungsform sind die Gurte vollständig von den einzelnen Bestandteilen lösbar.

In einer Ausführungsform umfasst die Stabilisierungsvorrichtung weiterhin Verbindungselemente. In einer Ausführungsform sind die Verbindungselemente als Gurte oder als Bänder oder als Gurtbänder ausgestaltet.

In einer Ausführungsform sind die Bestandteile und/oder die Verbindungselemente der Stabilisierungsvorrichtung individuell in variablen und unterschiedlichen Abmessungen gestaltbar. In einer Ausführungsform sind die Bestandteile und/oder die Verbindungselemente der Stabilisierungsvorrichtung miteinander kombinierbar. In einer Ausführungsform sind die Bestandteile und/oder die Verbindungselemente der Stabilisierungsvorrichtung individuell in variablen und unterschiedlichen Abmessungen gestaltbar und miteinander kombinierbar.

Vorteilhaft ist die Stabilisierungsvorrichtung damit an das jeweilige Tier und seine Anfordernisse und Bedürfnisse individuell angepasst. Es wird somit das Gewicht, die Größe und Anatomie, sowie die Belastbarkeit des Vierbeines berücksichtigt.

In einer bevorzugten Ausführungsform weist die Stabilisierungsvorrichtung einen modularen Aufbau auf. Unter einem modularen Aufbau wird die Kombinationsmöglichkeit unterschiedlich ausgestalteter Bestandteile und Verbindungselemente der Stabilisierungsvorrichtung verstanden.

Die erfindungsgemäße Stabilisierungsvorrichtung ist an das Tier einfach und schnell anlegbar und wieder abnehmbar ausgebildet.

Erfindungsgemäß umfasst die Stabilisierungsvorrichtung ein Bauchelement. Das Bauchelement ist bevorzugt im Bereich des Bauches des Tieres anordbar ausgebildet. Erfindungsgemäß wird unter dem Bauchbereich des Tieres das Abdomen verstanden.

Erfindungsgemäß ist das Bauchelement im Wesentlichen flächig ausgebildet. Unter einer im Wesentlichen flächigen Ausbildung des Bauchelementes wird im Sinne der Erfindung ein Bauchelement verstanden, welches großflächig Bereiche des Bauches abdeckt. In einer Ausführungsform werden mindestens 60%, bevorzugt mindestens 70%, ganz besonders bevorzugt mindestens 80% des Bauchbereiches des Tieres von dem Bauchelement abgedeckt.

In einer bevorzugten Ausführungsform weist das Bauchelement der Stabilisierungsvorrichtung eine Aussparung für das Geschlechtsorgan bei männlichen Tieren wie beispielsweise Rüden auf. In einer Ausführungsform weist das Bauchelement eine Aussparung für das Geschlechtsorgan bei männlichen Tieren wie beispielsweise Rüden auf. Vorteilhaft wird dadurch das Wasserlassen unkompliziert ermöglicht sowie der Tragekomfort der Stabilisierungsvorrichtung gesteigert.

Erfindungsgemäß weist das Bauchelement eine flexible und anatomisch anpassbare Auflagefläche auf. Im Sinne der Erfindung heißt anatomisch anpassbar, dass das Bauchelement sich an den Bauchbereich des Tieres flexibel anschmiegt. Dadurch wird der Tragekomfort gesteigert. Weiterhin vorteilhaft wird durch die Ausgestaltung der Auflagefläche beispielweise eine muskel-, gelenk- und blutgefäßschonende Behandlung des Tieres gewährleistet. Somit wird der Bewegungsapparat des Tieres geschont.

In einer Ausführungsform ist die Auflagefläche des Bauchelementes individuell an den Bauchbereich des Tieres angepasst. In einer Ausführungsform ist die Auflagefläche des Bauchelementes birnenförmig gestaltet.

Erfindungsgemäß sind mindestens vier Verbindungselemente am Bauchelement angeordnet. In einer alternativen Ausführungsform sind mindestens zwei Verbindungselemente am Bauchelement angeordnet.

Erfindungsgemäß sind mindestens vier Verbindungselemente mit daran angeordneten ersten Verschlussmitteln am Bauchelement angeordnet. In einer weiteren Ausführungsform sind vier Verbindungselemente am Bauchelement angeordnet. In einer Ausführungsform sind die Verbindungselemente punktuell, d.h. an verschiedenen Stellen des Bauchelementes angeordnet. In einer Ausführungsform sind die Verbindungselemente voneinander beabstandet am Bauchelement angeordnet. In einer Ausführungsform berühren sich die am Bauchelement angeordneten Verbindungselemente nicht. Vorteilhaft wird damit eine hohe Stabilität der Stabilisierungsvorrichtung erreicht.

In einer Ausführungsform sind die Verbindungselemente an dem Bauchelement nicht lösbar angeordnet verbunden, beispielsweise durch Vernähen.

In einer Ausführungsform sind die Verbindungselemente spiegelsymmetrisch zu der Längsachse des Bauchelementes am Bauchelement angeordnet. In einer bevorzugten Ausführungsform sind die mindestens vier Verbindungselemente spiegelsymmetrisch zu der Längsachse des Bauchelementes voneinander beabstandet am Bauchelement angeordnet. Die Längsachse des Bauchelementes stellt dabei am Bauchelement die fiktive zentral angeordnete Linie in Laufrichtung des Tieres dar. In einer bevorzugten Ausführungsform sind, auf die Längsachse bezogen, zwei Verbindungselemente auf der linken Seite des Bauchelementes und zwei Verbindungselemente auf der rechten Seite des Bauchelementes angeordnet. Vorteilhaft wird durch die symmetrische Anordnung der Verbindungselemente bei verbundenem Zustand von Bauch- und Rückenelement ein stabiler, fester und rutschsicherer Halt der Stabilisierungsvorrichtung gewährleistet.

In einer Ausführungsform umfasst die Stabilisierungsvorrichtung mindestens vier Verbindungselemente. In einer alternativen Ausführungsform umfasst die Stabilisierungsvorrichtung mindestens zwei Verbindungselemente. In einer bevorzugten Ausführungsform umfasst die Stabilisierungsvorrichtung vier Verbindungselemente.

In einer bevorzugten Ausführungsform sind an den mindestens vier Verbindungselementen des Bauchelementes erste Verschlussmittel angeordnet. In einer Ausführungsform sind an den mindestens vier Verbindungselementen des Bauchelementes zumindest vier erste Verschlussmittel angeordnet.

In einer Ausführungsform entspricht die Anzahl derVerbindungselemente des Bauchelementes der Anzahl der ersten Verschlussmittel. Vorteilhaft weist damit jedes Verbindungselement des Bauchelementes ein erstes Verschlussmittel auf. In einer Ausführungsform ist an jedem Verbindungselement des Bauchelementes ein erstes Verschlussmittel angeordnet. In einer Ausführungsform sind an den vier Verbindungselementen des Bauchelementes vier erste Verschlussmittel angeordnet. In einer alternativen Ausführungsform sind an den zwei Verbindungselementen des Bauchelementes zwei erste Verschlussmittel angeordnet.

In einer Ausführungsform umfassen die ersten Verschlussmittel des Bauchelementes mechanische Verschlussmittel wie Schnallen, insbesondere Rollschnallen, Doppelsteg-Schnallen, Dreisteg-Schnallen, Leiterschnallen, Schiebeschnallen, Klemmschnallen, Klickschnallen (Blitz- oder Klickverschluss, auch Klickschließen genannt), Steckschnallen oder Formschlussschnallen. In einer weiteren Ausführungsform umfassen die ersten Verschlussmittel des Bauchelementes mechanische Verschlussmittel wie Knebelverschlüsse, Klettverschlüsse, Druckknöpfe oder Reißverschlüsse. In einer weiteren Ausführungsform umfassen die ersten Verschlussmittel des Bauchelementes mechanische Verschlussmittel wie Steckschlösser. In einer weiteren Ausführungsform weisen die ersten Verschlussmittel des Bauchelementes Verstellschieber auf, wodurch vorteilhaft die Länge der Verbindungselemente zum Rückenelement eingestellt werden kann.

In einer Ausführungsform sind die Verbindungselemente zumindest teilweise an den Rändern des Bauchelementes angeordnet. In einer weiteren Ausführungsform sind die Verbindungselemente spiegelsymmetrisch zu der Längsachse des Bauchelementes an den Rändern des Bauchelementes angeordnet. In einer weiteren Ausführungsform sind alle Verbindungselemente an den Rändern des Bauchelementes angeordnet.

In einer Ausführungsform umfasst die Stabilisierungsvorrichtung ein Rückenelement. Erfindungsgemäß ist das Rückenelement im Bereich des Rückens des Tieres anordbar ausgebildet.

Unter dem Rückenelement wird beispielsweise auch ein Rückenteil verstanden.

Erfindungsgemäß ist das Rückenelement flächig ausgebildet. Unter einer flächigen Ausbildung des Bauchelementes wird im Sinne der Erfindung ein Rückenelement verstanden, welches großflächig Bereiche des Rückens abdeckt.

In einer Ausführungsform ist das Rückenelement stabilisierend ausgebildet. In einer Ausführungsform ist das Rückenelement im Bereich der Wirbelsäule stabilisierend und starr ausgebildet. Vorteilhaft wird damit die Wirbelsäule gestützt und eine Durchbiegung vermieden. In einer Ausführungsform ist die stabilisierende Ausbildung des Rückenelementes durch eine stabilisierende Einlage gewährleistet.

In einer Ausführungsform ist die Auflagefläche des Rückenelementes individuell an den Rückenbereich des Tieres angepasst.

Im Sinne der Erfindung weist das Rückenelement zumindest abschnittsweise zu der Längsachse mindestens ein Mittel zur Stabilisierung auf. Unter dem Mittel zur Stabilisierung wird im Sinne der Erfindung beispielsweise auch eine Rückenplatte verstanden.

In einer Ausführungsform weist das Rückenelement zumindest abschnittsweise zu der Längsachse ein Mittel zur Stabilisierung auf. In einer alternativen Ausführungsform weist das Rückenelement zumindest abschnittsweise zu der Längsachse mehrere Mittel zur Stabilisierung auf. Vorteilhaft sind die Mittel zur Stabilisierung dabei parallel voneinander beabstandet angeordnet, um eine gute Stabilität des Rückenelementes zu erreichen.

In einer Ausführungsform verläuft das Mittel zur Stabilisierung durchgehend zu der Längsachse des Rückenelementes. In einer alternativen Ausführungsform verlaufen mehrere Mittel zur Stabilisierung durchgehend zu der Längsachse des Rückenelementes. Vorteilhaft sind die Mittel zur Stabilisierung dabei parallel zu der Längsachse und voneinander beabstandet angeordnet, um eine gute Stabilität des Rückenelementes zu erreichen.

Die Längsachse des Rückenelementes stellt dabei am Rückenelement die fiktive zentral angeordnete Linie in Laufrichtung des Tieres dar. Die Längsachse des Rückenelementes verläuft entlang der Wirbelsäule des Tieres.

In einer weiteren Ausführungsform ist das Mittel zur Stabilisierung im Rückenelement eine Platte oder eine Schiene. In einer Ausführungsform wird das Mittel zur Stabilisierung des Rückenelementes in eine dafür vorgesehene Aussparung an der Oberseite des Rückenelementes eingebracht. In einer weiteren Ausführungsform wird das Mittel zur Stabilisierung des Rückenelementes im Rückenelement befestigt, beispielsweise durch Vernähen. In einer weiteren Ausführungsform wird das Mittel zur Stabilisierung des Rückenelementes unter einem Verbindungselement des Rückenelementes vernäht.

In einer Ausführungsform ist das Mittel zur Stabilisierung im Rückenelement eine Metallschiene oder eine Kunststoffschiene, wobei durch die Kunststoffschiene vorteilhaft das Gewicht des Rückenelementes reduziert wird.

Eine weitere Stabilisierung des Rückenelementes wird durch Einlagen und Nähte erreicht.

In einer Ausführungsform weist das Rückenelement für die ersten Verschlussmittel des Bauchelementes korrespondierende zweite Verschlussmittel auf. In einer Ausführungsform stellen die zweiten Verschlussmittel die korrespondierenden Gegenstücke zu den ersten Verschlussmitteln dar, sodass vorteilhaft die ersten und zweiten Verschlussmittel formschlüssig und lösbar miteinander verbindbar ausgebildet sind.

In einer Ausführungsform bestehen die ersten und die zweiten Verschlussmittel aus dem gleichen Material. In einer alternativen Ausführungsform bestehen die ersten und die zweiten Verschlussmittel aus unterschiedlichen Materialien. Als Materialien für die ersten und die zweiten Verschlussmittel werden bspw. Metalle oder vorteilhaft Kunststoffe verwendet.

Erfindungsgemäß entspricht die Anzahl der ersten Verschlussmittel des Bauchelementes der Anzahl der korrespondierenden zweiten Verschlussmittel des Rückenelementes. In einer Ausführungsform sind für die ersten Verschlussmittel des Bauchelementes zumindest vier korrespondierende zweite Verschlussmittel an dem Rückenelement angeordnet. In einer weiteren Ausführungsform sind für die ersten Verschlussmittel des Bauchelementes vier korrespondierende zweite Verschlussmittel an dem Rückenelement angeordnet.

Die korrespondierenden zweiten Verschlussmittel sind dabei vorteilhaft so an dem Rückenelement angeordnet, dass diese mit den ersten Verschlussmitteln des Bauchelementes für den Benutzer einfach zu verbinden sind. Sowohl die ersten Verschlussmittel als auch die korrespondierenden zweiten Verschlussmittel sind so an dem Bauch- bzw. Rückenelement angeordnet, dass diese für den Benutzer einfach zugänglich sind.

In einer Ausführungsform ist das Bauchelement mit dem Rückenelement lösbar verbindbar ausgebildet.

Erfindungsgemäß ist das Bauchelement mit dem Rückenelement durch die am Bauchelement befindlichen Verbindungselemente mit den daran angeordneten ersten Verschlussmitteln und den korrespondierenden zweiten Verschlussmitteln, welche am Rückenelement angeordnet sind, lösbar verbindbar ausgebildet. Das Verschlussmittel des Bauchelementes ist dabei mechanisch lösbar in das korrespondierende zweite Verschlussmittel des Rückenelementes verbindbar ausgebildet.

In einer weiteren Ausführungsform sind die Verbindungselemente des Bauchelementes und die korrespondierenden zweiten Verschlussmittel des Rückenelementes als Klettverschlüsse ausgebildet, welche lösbar miteinander verbindbar sind.

Werden das Bauchelement und das Rückenelement miteinander verbunden, verlaufen die mindestens vier Verbindungselemente in einer bevorzugten Ausführungsform entlang anatomisch geeigneter Positionen des Rumpfes des Tieres. In einer alternativen Ausführungsform verlaufen die mindestens zwei Verbindungselemente in einer bevorzugten Ausführungsform entlang anatomisch geeigneter Positionen des Rumpfes des Tieres, wenn das Bauchelement und das Rückenelement miteinander verbunden werden.

Unter anatomisch geeigneten Positionen sind erfindungsgemäß jene Positionen zu verstehen, welche das Tier in seiner Bewegung nicht hindern oder schmerzen. Dazu zählt beispielsweise der Verlauf der Verbindungselemente entlang geeigneter Rippen, insbesondere im Bereich der dritten, vierten oder fünften Rippe. Vorteilhaft wird durch den Verlauf der Verbindungselemente im Bereich der dritten, vierten oder fünften Rippe die höchste Stabilität beim Anliegen der Stabilisierungsvorrichtung erreicht. Weiterhin verlaufen die Verbindungselemente im verbundenen Zustand zwischen Bauchelement und Rückenelement muskelentlastend und klemmen keine Blutgefäße ab.

In einer Ausführungsform weist die Stabilisierungsvorrichtung mindestens ein lösbares Trageelement auf. Vorteilhaft kann das Tier dadurch angehoben oder getragen und transportiert werden. In einer bevorzugten Ausführungsform ist in die ersten Aufnahmeeinrichtungen mindestens ein lösbares Trageelement anbringbar.

In einer Ausführungsform ist das Trageelement mit der Stabilisierungsvorrichtung lösbar verbunden. In einer Ausführungsform ist das Trageelement mit der Stabilisierungsvorrichtung über erste Aufnahmeeinrichtungen lösbar angeordnet verbunden.

In einer bevorzugten Ausführungsform weist die Stabilisierungsvorrichtung mindestens zwei erste Aufnahmeeinrichtungen auf.

In einer bevorzugten Ausführungsform sind die mindestens zwei ersten Aufnahmeeinrichtungen am Rückenelement und/oder am Schulterelement der Stabilisierungsvorrichtung angeordnet. In einer Ausführungsform weist das Rückenelement und/oder das Schulterelement mehr als zwei voneinander beabstandete erste Aufnahmeeinrichtungen auf.

In einer Ausführungsform sind die mindestens zwei ersten Aufnahmeeinrichtungen voneinander beabstandet angeordnet. In einer Ausführungsform sind die mindestens zwei ersten Aufnahmeeinrichtungen zu der Längsachse des Rückenelementes und/oder zu der Längsachse des Schulterelementes und somit zu der Wirbelsäule angeordnet.

In einer Ausführungsform sind die mindestens zwei ersten Aufnahmeeinrichtungen nicht lösbar mit dem Rückenelement und/oder dem Schulterelement verbunden. In einer Ausführungsform ist eine erste Aufnahmeeinrichtung nicht lösbar mit dem Rückenelement und/oder dem Schulterelement verbunden. In einer Ausführungsform ist dazu ein Teil des Rückenelementes und/oder des Schulterelementes als Gurtband ausgebildet, wobei das Gurtband so vernäht ist, dass es eine Lasche aufweist, in welches die erste Aufnahmeeinrichtung einsetzbar ist. Das Gurtband wiederum ist mit dem Rückenelement und/oder dem Schulterelement nicht lösbar verbunden, beispielsweise durch Vernähen und liegt dicht am Rückenelement und/oder am Schulterelement an.

In einer Ausführungsform sind zwei erste Aufnahmeeinrichtungen jeweils nicht lösbar am Rückenelement der Stabilisierungsvorrichtung angeordnet. In einer weiteren Ausführungsform sind zwei erste Aufnahmeeinrichtungen jeweils nicht lösbar am Schulterelement der Stabilisierungsvorrichtung angeordnet. In einer alternativen Ausführungsform sind jeweils mindestens eine erste Aufnahmeeinrichtung am Rückenelement und mindestens eine erste Aufnahmeeinrichtung am Schulterelement der Stabilisierungsvorrichtung jeweils nicht lösbar angeordnet. In einer alternativen Ausführungsform sind jeweils eine erste Aufnahmeeinrichtung am Rückenelement und eine erste Aufnahmeeinrichtung am Schulterelement der Stabilisierungsvorrichtung jeweils nicht lösbar angeordnet.

In einer Ausführungsform sind die zwei ersten Aufnahmeeinrichtungen an den beiden Enden zu der Längsachse des Rückenelementes und/oder des Schulterelementes jeweils nicht lösbar angeordnet. In einer Ausführungsform sind zwei erste Aufnahmeeinrichtungen an den beiden Enden zu der Längsachse des Rückenelementes und zwei erste Aufnahmeeinrichtungen an den beiden Enden zu der Längsachse des Schulterelementes jeweils nicht lösbar angeordnet.

In einer weiteren alternativen Ausführungsform sind zwei erste Aufnahmeeinrichtungen am Rückenelement und eine erste Aufnahmeeinrichtung am Schulterelement jeweils nicht lösbar angeordnet. Vorteilhaft lässt sich durch diese Anordnungen das Trageelement variierend einbringen, sodass sich das Tier entweder am Hinterteil oder komplett anheben lässt.

In einer weiteren alternativen Ausführungsform sind eine erste Aufnahmeeinrichtung am Rückenelement und zwei erste Aufnahmeeinrichtungen am Schulterelement jeweils nicht lösbar angeordnet. Vorteilhaft lässt sich durch diese Anordnungen das Trageelement variierend einbringen, sodass sich das Tier entweder am Vorderteil oder komplett anheben lässt.

In einer weiteren alternativen Ausführungsform sind zwei erste Aufnahmeeinrichtungen am Rückenelement und zwei erste das Trageelement am Schulterelement jeweils nicht lösbar angeordnet. Vorteilhaft lässt sich durch diese Anordnungen das Trageelement variierend einbringen, sodass sich das Tier entweder am Hinterteil oder am Vorderteil oder komplett anheben lässt.

In einer Ausführungsform sind die ersten Aufnahmeeinrichtungen der Stabilisierungsvorrichtung als Ringe, insbesondere O-Ringe, Ösen oder D-Ringe zur Befestigung der Tragegriffe, ausgebildet. In einer Ausführungsform ist die erste Aufnahmeeinrichtung aus einem stabilen Material wie Metall, einer Metalllegierung oder Kunststoff hergestellt.

In einer Ausführungsform ist das Trageelement als Griff ausgebildet. In einer weiteren Ausführungsform ist das Trageelement als Tragegriff oder als Trageband ausgebildet, wobei das Trageband vorteilhaft elastischer ausgebildet ist. In einer Ausführungsform weist das Trageelement zwei Enden auf. Das Trageelement ist vorteilhaft aus Kunststoff oder einem robusten textilen Material hergestellt.

In einer Ausführungsform weist das Trageelement mindestens eine lösbare Verbindungsmöglichkeit in Form einer zweiten Aufnahmeeinrichtung auf, wobei die zweite Aufnahmeeinrichtung an die erste Aufnahmeeinrichtung verbindbar ausgebildet ist. In einer Ausführungsform weist das Trageelement zwei lösbare Verbindungsmöglichkeiten in Gestalt von zwei zweiten Aufnahmeeinrichtungen an die ersten Aufnahmeeinrichtungen auf.

In einer bevorzugten Ausführungsform weist das lösbare Trageelement mindestens zwei zweite Aufnahmeeinrichtungen auf. In einer Ausführungsform weist das Trageelement an seinen beiden Enden jeweils eine zweite Aufnahmeeinrichtung auf, welche in jeweils eine erste Aufnahmeeinrichtung am Rückenelement und/oder am Schulterelement lösbar verbindbar ausgebildet ist. In einer Ausführungsform sind somit die ersten Aufnahmeeinrichtungen lösbar mit zweiten Aufnahmeeinrichtungen verbindbar, wodurch Trageelemente an die Stabilisierungsvorrichtung anbringbar sind. In einer Ausführungsform sind die zweiten Aufnahmeeinrichtungen als Haken wie beispielsweise als Karabinerhaken oder als Schnappverschlüsse ausgeführt. Das lösbare Verbinden erfolgt beispielsweise durch Einhaken der zweiten Aufnahmeeinrichtung in die erste Aufnahmeeinrichtung.

In einer Ausführungsform ist das Trageelement aus einem flexiblen und dehnfähigen Material gestaltet. Vorteilhaft kann der Halter beim Anheben des Hundes oder beim Gassi gehen in aufrechter Position bleiben, wodurch der eigene Rücken geschont wird.

In einer bevorzugten Ausführungsform umfasst die Stabilisierungsvorrichtung ein Vorderteil. In einer bevorzugten Ausführungsform weist das Vorderteil ein Brustelement auf. Das Brustelement ist bevorzugt im Bereich der Brust des Tieres anordbar ausgebildet.

Unter dem Brustelement wird beispielsweise auch ein Brustteil verstanden.

In einer bevorzugten Ausführungsform ist das Brustelement flächig ausgebildet. Unter einer flächigen Ausbildung des Brustelementes wird im Sinne der Erfindung ein Brustelement verstanden, welches großflächig Bereiche der Brust abdeckt.

In einer Ausführungsform weist das Brustelement eine flexible und anatomisch anpassbare Auflagefläche auf. Im Sinne der Erfindung heißt anatomisch anpassbar, dass das Brustelement sich an den Brustbereich des Tieres flexibel anschmiegt. Dadurch wird der Tragekomfort gesteigert. Weiterhin vorteilhaft wird durch die Ausgestaltung des Brustelementes beispielweise eine muskel-, gelenk- und blutgefäßschonende Behandlung des Tieres gewährleistet. Somit wird der Bewegungsapparat des Tieres geschont.

In einer Ausführungsform ist die Auflagefläche des Brustelementes individuell an den Brustbereich des Tieres angepasst. In einer Ausführungsform ist die Auflagefläche des Brustelementes in der Draufsicht stimmgabelförmig gestaltet, wobei die beiden Enden der Zinken der Stimmgabel um den Hals des Tieres verlaufen.

In einer Ausführungsform sind mindestens zwei Verbindungselemente am Brustelement angeordnet. In einer weiteren Ausführungsform sind zwei Verbindungselemente am Brustelement angeordnet. In einer Ausführungsform sind die Verbindungselemente punktuell, d.h. an verschiedenen Stellen des Brustelementes angeordnet. In einer Ausführungsform sind die Verbindungselemente voneinander beabstandet am Brustelement angeordnet. In einer Ausführungsform berühren sich die am Brustelement angeordneten Verbindungselemente nicht. Vorteilhaft wird damit eine hohe Stabilität der Stabilisierungsvorrichtung erreicht.

In einer Ausführungsform sind die Verbindungselemente an dem Brustelement nicht lösbar angeordnet verbunden, beispielsweise durch Vernähen.

In einer Ausführungsform sind die Verbindungselemente spiegelsymmetrisch zu der Längsachse des Brustelementes am Brustelement angeordnet. Die Längsachse des Brustelementes stellt dabei am Brustelement die fiktive zentral angeordnete Linie in Laufrichtung des Tieres dar. In einer bevorzugten Ausführungsform ist, auf die Längsachse bezogen, ein Verbindungselement auf der linken Seite des Brustelementes und ein Verbindungselement auf der rechten Seite des Brustelementes angeordnet. Vorteilhaft wird durch die symmetrische Anordnung der Verbindungselemente bei verbundenem Zustand von Brust- und Schulterelementes ein fester und rutschsicherer Halt der Stabilisierungsvorrichtung gewährleistet.

In einer Ausführungsform sind an den mindestens zwei Verbindungselementen des Brustelementes erste Verschlussmittel angeordnet. In einer Ausführungsform sind an den mindestens zwei Verbindungselementen des Brustelementes mindestens zwei erste Verschlussmittel angeordnet.

In einer Ausführungsform entspricht die Anzahl der Verbindungselemente des Brustelementes der Anzahl der ersten Verschlussmittel des Brustelementes. Vorteilhaft weist damit jedes Verbindungselement des Brustelementes ein erstes Verschlussmittel auf. In einer Ausführungsform sind an den zwei Verbindungselementen des Brustelementes zwei erste Verschlussmittel angeordnet. In einer Ausführungsform ist an jedem Verbindungselement des Brustelementes ein erstes Verschlussmittel angeordnet.

In einer Ausführungsform umfassen die ersten Verschlussmittel des Brustelementes mechanische Verschlussmittel wie Schnallen, insbesondere Rollschnallen, Doppelsteg-Schnallen, Dreisteg-Schnallen, Leiterschnallen, Schiebeschnallen, Klemmschnallen, Klickschnallen (Blitz- oder Klickverschluss, auch Klickschließen genannt), Steckschnallen oder Formschlussschnallen. In einer weiteren Ausführungsform umfassen die ersten Verschlussmittel des Brustelementes mechanische Verschlussmittel wie Knebelverschlüsse, Klettverschlüsse, Druckknöpfe oder Reißverschlüsse. In einer weiteren Ausführungsform umfassen die ersten Verschlussmittel des Brustelementes mechanische Verschlussmittel wie Steckschlösser. In einer weiteren Ausführungsform weisen die ersten Verschlussmittel des Brustelementes Verstellschieber auf, wodurch vorteilhaft die Länge der Verbindungselemente zum Schulterelement eingestellt werden kann.

In einer Ausführungsform sind die Verbindungselemente zumindest teilweise an den Rändern des Brustelementes angeordnet. In einer weiteren Ausführungsform sind die Verbindungselemente spiegelsymmetrisch zu der Längsachse des Brustelementes an den Rändern des Brustelementes angeordnet. In einer weiteren Ausführungsform sind alle Verbindungselemente an den Rändern des Brustelementes angeordnet.

In einer bevorzugten Ausführungsform umfasst die Stabilisierungsvorrichtung ein Vorderteil. In einer bevorzugten Ausführungsform weist das Vorderteil ein Schulterelement auf. Das Schulterelement ist bevorzugt im Bereich der Schulter des Tieres anordbar ausgebildet.

Unter dem Schulterelement wird beispielsweise auch ein Schulterteil verstanden.

In einer bevorzugten Ausführungsform ist das Schulterelement flächig ausgebildet. Unter einer im Wesentlichen flächigen Ausbildung des Schulterelementes wird im Sinne der Erfindung ein Schulterelement verstanden, welches großflächig Bereiche der Schulter abdeckt.

In einer Ausführungsform ist das Schulterelement stabilisierend ausgebildet. In einer Ausführungsform ist das Schulterelement im Bereich der Wirbelsäule stabilisierend und starr ausgebildet. Vorteilhaft wird damit die Wirbelsäule gestützt und eine Verbiegung derselben vermieden.

In einer Ausführungsform ist die Auflagefläche des Schulterelementes individuell an den Schulterbereich des Tieres angepasst. In einer Ausführungsform ist die Auflagefläche des Schulterelementes in der Draufsicht wie eine Stimmgabel gestaltet, wobei die beiden Enden der Zinken der Stimmgabel um den Hals des Tieres verlaufen.

In einer Ausführungsform weist das Schulterelement für die ersten Verschlussmittel des Brustelementes korrespondierende zweite Verschlussmittel auf. In einer Ausführungsform entspricht die Anzahl der korrespondierenden zweiten Verschlussmittel des Schulterelementes der Anzahl der ersten Verschlussmittel des Brustelementes. In einer Ausführungsform sind für die ersten Verschlussmittel des Brustelementes mindestens zwei zweite korrespondierende Verschlussmittel an dem Schulterelement angeordnet. In einer weiteren Ausführungsform sind für die ersten Verschlussmittel des Brustelementes zwei zweite korrespondierende Verschlussmittel an dem Schulterelement angeordnet.

Die korrespondierenden zweiten Verschlussmittel sind dabei so an dem Schulterelement angeordnet, dass die ersten Verschlussmittel des Brustelementes für den Benutzer einfach zu verbinden sind. Sowohl die ersten Verschlussmittel als auch die korrespondierenden zweiten Verschlussmittel sind so an dem Brust- bzw. Schulterelement angeordnet, dass diese für den Benutzer einfach zugänglich sind.

In einer Ausführungsform weist das Schulterelement zumindest abschnittsweise zu der Längsachse ein Mittel zur Stabilisierung auf. In einer alternativen Ausführungsform weist das Schulterelement zumindest abschnittsweise zu der Längsachse mehrere Mittel zur Stabilisierung auf. Vorteilhaft sind die Mittel zur Stabilisierung dabei parallel und voneinander beabstandet angeordnet, um eine gute Stabilität des Schulterelementes zu erreichen.

In einer weiteren Ausführungsform verläuft das Mittel zur Stabilisierung durchgehend zu der Längsachse des Schulterelementes. In einer alternativen Ausführungsform verlaufen mehrere Mittel zur Stabilisierung durchgehend zu der Längsachse des Schulterelementes. Vorteilhaft sind die Mittel zur Stabilisierung dabei parallel und voneinander beabstandet angeordnet, um eine gute Stabilität des Schulterelementes zu erreichen.

Die Längsachse des Schulterelementes stellt dabei am Schulterelement die fiktive zentral angeordnete Linie in Laufrichtung des Tieres dar. Die Längsachse des Schulterelementes verläuft entlang der Wirbelsäule des Tieres.

In einer Ausführungsform ist das Mittel zur Stabilisierung im Schulterelement eine Platte oder eine Schiene. In einer Ausführungsform wird das Mittel zur Stabilisierung des Schulterelementes in eine dafür vorgesehene Aussparung an der Oberseite des Schulterelementes geschoben. In einer weiteren Ausführungsform wird das Mittel zur Stabilisierung des Schulterelementes im Schulterelement befestigt, beispielsweise durch Vernähen. In einer weiteren Ausführungsform wird das Mittel zur Stabilisierung des Schulterelementes unter einem Verbindungselement des Schulterelementes vernäht.

In einer Ausführungsform ist das Mittel zur Stabilisierung im Schulterelement eine Metallschiene. In einer weiteren Ausführungsform ist das Mittel zur Stabilisierung im Schulterelement eine Kunststoffschiene, wodurch vorteilhaft das Gewicht des Schulterelementes reduziert wird.

Eine weitere Stabilisierung des Schulterelementes wird durch Einlagen und Nähte erreicht.

In einer Ausführungsform ist das Brustelement mit dem Schulterelement über mindestens eine lösbare Verbindung verbunden.

Unter der lösbaren Verbindung von dem Brustelement mit dem Schulterelement wird beispielsweise auch eine Naht zur Verbindung von Schulter- und Brustelement verstanden.

In einer Ausführungsform ist das Brustelement mit dem Schulterelement durch die am Brustelement befindlichen Verbindungselemente mit den daran angeordneten ersten Verschlussmitteln und den korrespondierenden zweiten Verschlussmittel, welche am Schulterelement angeordnet sind, lösbar verbindbar ausgebildet. In einer bevorzugten Ausführungsform ist das Brustelement mit dem Schulterelement durch mindestens zwei Verbindungselemente verbunden. Das erste Verschlussmittel des Brustelementes ist dabei mechanisch lösbar in das korrespondierende zweite Verschlussmittel des Schulterelementes verbindbar ausgebildet.

In einer weiteren Ausführungsform sind die Verbindungselemente des Brustelementes und die zu den ersten Verschlussmitteln korrespondierenden zweiten Verschlussmittel des Schulterelementes als Klettverschlüsse ausgebildet, welche lösbar miteinander verbindbar sind.

Werden das Brustelement und das Schulterelement miteinander verbunden, verlaufen in einer Ausführungsform die Verbindungselemente entlang anatomisch geeigneter Positionen des Rumpfes des Tieres. Unter anatomisch geeigneten Positionen sind erfindungsgemäß jene Positionen zu verstehen, welche das Tier in seiner Bewegung nicht hindern oder schmerzen. Dazu zählt beispielsweise der Verlauf der Verbindungselemente im Bereich geeigneter Rippen, insbesondere im Bereich der dritten, vierten und fünften Rippe. Weiterhin verlaufen die Verbindungselemente im verbundenen Zustand zwischen Brustelement und Schulterelement muskelentlastend und klemmen keine Blutgefäße ab.

In einer bevorzugten Ausführungsform umfasst die Stabilisierungsvorrichtung eine Vorrichtung zur Erfassung von Vitalfunktionen von Tieren und/oder zur Ortung und/oder Überwachung und/oder Geodatenübermittlung von Tieren.

In einer Ausführungsform weisen zumindest einige der Bestandteile und/oder einige der Verbindungselemente der Stabilisierungsvorrichtung textile Materialien auf.

In einer Ausführungsform weisen die Bestandteile der Stabilisierungsvorrichtung eine Außenschicht auf. In einer weiteren Ausführungsform weisen die Bestandteile der Stabilisierungsvorrichtung eine Innenschicht auf. Die Außenschicht entspricht der Schicht, welche in Kontakt mit der Umgebung des Tieres ist. Die Innenschicht entspricht der Schicht, welche in Kontakt mit der Haut oder dem Fell des Tieres ist. In einer weiteren Ausführungsform weisen die Bestandteile der Stabilisierungsvorrichtung eine Zwischenschicht auf. Die Zwischenschicht entspricht einer Füllung, welche zwischen Außenschicht und Innenschicht angeordnet ist.

In einer Ausführungsform umfassen alle textile Materialien aufweisenden Teile der Stabilisierungsvorrichtung dasselbe textile Material. Vorteilhaft handelt es sich um ein robustes textiles Material. In einer alternativen Ausführungsform umfassen alle textile Materialien aufweisenden Teile der Stabilisierungsvorrichtung sich unterscheidende textile Materialien.

In einer Ausführungsform ist das robuste textile Material ein textiles Gewebe oder Stoff. In einer Ausführungsform ist das robuste textile Material ein textiles Gewebe aus Fasern aus Kunst- und/oder Naturfasern. In einer Ausführungsform ist das robuste textile Material ein textiles Gewebe aus Polyamidfasern. In einer Ausführungsform ist das robuste textile Material ein textiles Gewebe aus Polyamidfasern und verschiedenen weiteren Kunst- oder Naturfasern. In einer Ausführungsform ist das robuste textile Material ein textiles Material, welche auch als Außenstoff von Koffern verwendet und im Folgenden als Kofferstoff bezeichnet wird. Das robuste textile Material der Stabilisierungsvorrichtung zeichnet sich durch vorteilhafte Eigenschaften aus: So ist das robuste textile Material schmutzabweisend, waschbar und atmungsaktiv. Vorteilhaft trocknet das robuste textile Material schnell, ist strapazierfähig und leicht zu reinigen.

In einer Ausführungsform weist das robuste textile Material eine Wassersäule von 3000 bis 4000 mmH₂O, bevorzugt 3500 mmH₂O auf. In einer weiteren Ausführungsform weist das robuste textile Material der Stabilisierungsvorrichtung Zugfestigkeiten im Bereich von 1 bis 5 kg/cm auf. Vorteilhaft wird die Zugfestigkeit des robusten textilen Materials in Abhängigkeit von der Größe des Hundes und somit von der Größe den jeweiligen Bauch-, Brust-, Schulter- und Rückenelementen gewählt.

In einer Ausführungsform weisen zumindest einige der Bestandteile der Stabilisierungsvorrichtung eine Zwischenschicht auf. In einer Ausführungsform weisen das Bauchelement und/oder das Brustelement und/oder das Schulterelement und/oder das Rückenelement eine Zwischenschicht auf. In einer weiteren Ausführungsform weist die Auflagefläche von Bauchelement und/oder Brustelement eine Zwischenschicht auf.

In einer Ausführungsform ist die Zwischenschicht ein textiles Material. In einer Ausführungsform ist die Zwischenschicht als textiles Gewebe ausgebildet und in das textile Material der Außen- und/oder Innenschicht eingebracht, beispielsweise eingewebt. In einer Ausführungsform ist die Zwischenschicht als Abstandsgewirke ausgebildet. Dabei ist das Abstandsgewirke unelastisch ausgebildet.

In einer bevorzugten Ausführungsform weist zumindest ein Teil der Bestandteile der Stabilisierungsvorrichtung ein unelastisches Abstandsgewirke auf. In einer Ausführungsform besteht das unelastische Abstandsgewirke zumindest teilweise aus Baumwolle. In einer weiteren bevorzugten Ausführungsform ist das Abstandsgewirke unelastisch ausgebildet und weist eine geringe Dehnfähigkeit auf. In einer bevorzugten Ausführungsform liegt die Dehnfähigkeit des unelastischen Abstandsgewirkes zwischen 0% und 15%, bevorzugt zwischen 0% und 10%, besonders bevorzugt zwischen 0% und 5%, ganz besonders bevorzugt um 0%. Vorteilhaft wird durch das Abstandsgewirke die Stabilität des Bauchelementes, Rückenelementes, Schulterelementes und/oder Brustelementes garantiert.

Vorteilhaft können durch die hohe Stabilität des unelastischen Abstandsgewirkes die vom Bauchelement abgehenden Verbindungselemente punktuell und nicht durchgängig angeordnet sein. Durch die hohe Stabilität des unelastischen Abstandsgewirkes kann weiterhin vorteilhaft auf durchgängige Verbindungselemente, welche das Bauchelement durchziehen, verzichtet werden. Vorteilhaft ist damit der Tragekomfort für das Tier erhöht und es werden keine Körperteile, Organe, Muskeln oder Blutgefäße im Bauchbereich bzw. im Bereich, in welchem das Abstandsgewirke eingebracht ist, abgeschnürt, abgedrückt oder verletzt.

In einer Ausführungsform sind die Bestandteile der Stabilisierungsvorrichtung zumindest teilweise gepolstert. In einer Ausführungsform sind das Bauchelement und/oder das Rückenelement und/oder das Brustelement und/oder das Schulterelement zumindest teilweise gepolstert. In einer Ausführungsform weist das Brustelement ein Brustbeinpolster auf.

In einer Ausführungsform sind die Bestandteile der Stabilisierungsvorrichtung gepolstert. In einer Ausführungsform sind das Bauchelement und/oder das Rückenelement und/oder das Brustelement und/oder das Schulterelement gepolstert. In einer Ausführungsform weist das Brustelement ein Brustbeinpolster auf.

In einer weiteren Ausführungsform sind die Verbindungselemente gepolstert. In einer weiteren Ausführungsform sind die Bestandteile und die Verbindungselemente der Stabilisierungsvorrichtung gepolstert. In einer weiteren Ausführungsform sind zumindest einige der Bestandteile und die Verbindungselemente der Stabilisierungsvorrichtung gepolstert.

In einer Ausführungsform bestehen die Verbindungselemente der Bestandteile bzw. die Verbindungen des lösbaren Verbindungsmittels aus einer Mischung von Polypropylen und Polyester, bevorzugt aus einer Mischung von 83% Polypropylen und 17% Polyester. Diese weisen eine Höchstzugkraft von mindestens 4500 N auf (ca. 450 kg) und sind ca. 25 mm breit, ca. 1,19 mm dick und weisen ein Gewicht von ca. 1,31 kg/100 m auf.

In einer alternativen Ausführungsform bestehen die Verbindungselemente der Bestandteile bzw. die Verbindungen des lösbaren Verbindungsmittels aus Polyester, bevorzugt aus 100% Polyester. Diese weisen eine Höchstzugkraft von mindestens 7500 N auf (ca. 750 kg) und sind ca. 40 mm breit, ca. 1,80 mm dick und weisen ein Gewicht von ca. 4,62 kg/100 m auf.

In einer Ausführungsform bestehen die ersten und zweiten Verschlussmittel der Bestandteile der Stabilisierungsvorrichtung bzw. die ersten und zweiten Verschlussmittel des lösbaren Verbindungsmittels der Stabilisierungsvorrichtung aus Metall oder Kunststoff.

In einer Ausführungsform bestehen die Steckschlösser aus Polyamid, bevorzugt Polyamid 6.6, weisen eine Festigkeit von 160 kg, 280 kg oder 600 kg und eine Durchlassweite von 40 mm auf. Weiterhin weisen manche Steckschlösser eine Verstellmöglichkeit sowie eine Festigkeit von 105 kg und eine Durchlassweite von 40 mm auf.

In einer Ausführungsform bestehen die Karabinerhaken aus Metall oder Kunststoff. Vorzugsweise bestehen die Karabinerhaken aus Acetal, und weisen eine Festigkeit von 35 kg und eine Durchlassweite von 25 mm auf.

In einer Ausführungsform bestehen die Verstellschieber aus Metall oder Kunststoff. In einer weiteren Ausführungsform bestehen die Verstellschieber aus Nylon und weisen eine Durchlassweite von 40 mm auf. Die Verstellschieber werden nicht direkt zugbelastet, da die Lastverteilung durch die Verbindungselemente der Bestandteile sowie durch die Verbindung des lösbaren Verbindungsmittels erfolgt.

In einer bevorzugten Ausführungsform ist in mindestens einem Bestandteil der Stabilisierungsvorrichtung eine Temperiervorrichtung angebracht.

In einer Ausführungsform ist die Temperiervorrichtung beheizbar und/oder kühlbar ausgebildet.

In einer bevorzugten Ausführungsform ist in mindestens einem Bestandteil der Stabilisierungsvorrichtung zumindest teilweise eine Temperiervorrichtung angebracht. In einer Ausführungsform ist in das Bauchelement und/oder das Rückenelement und/oder das Brustelement und/oder das Schulterelement zumindest teilweise eine Temperiervorrichtung eingebracht.

In einer Ausführungsform ist die Temperiervorrichtung in die Zwischenschicht der Bestandteile der Stabilisierungsvorrichtung eingebracht. In einer Ausführungsform umfasst die Temperiervorrichtung ein Peltier-Element, wodurch ein Heizen oder Kühlen der Stabilisierungsvorrichtung möglich ist. In einer weiteren Ausführungsform umfasst die Temperiervorrichtung Heizdrähte und/oder Heizkissen und/oder Kühlakkus und/oder Kühlgele. In einer Ausführungsform kann das Tier durch eine Temperiervorrichtung, welche beheizbar und/oder kühlbar ist, vorteilhaft gewärmt und/oder vor Unterkühlung geschützt werden. Durch die beheizbare Temperiervorrichtung kann das Tier weiterhin vorteilhaft in einem Transportmittel auch bei niedrigeren Temperaturen gehalten werden.

In einer Ausführungsform kann das Tier durch eine kühlbare Temperiervorrichtung vorteilhaft gekühlt und/oder vor Überhitzung geschützt werden. Durch die kühlbare Temperiervorrichtung kann das Tier weiterhin vorteilhaft in einem Transportmittel auch bei höheren Temperaturen gehalten werden.

In einer Ausführungsform erfolgt das Heizen oder Kühlen der Stabilisierungsvorrichtung über elektrische und/oder chemische, exotherm ablaufende Prozesse.

In einer Ausführungsform stammt die Energie für die Temperiervorrichtung von einem Energiespeicher, welcher in der Stabilisierungsvorrichtung angebracht ist.

In einer Ausführungsform bilden das Bauchelement mit den Verbindungselementen und den daran angeordneten ersten Verschlussmitteln sowie das Rückenelement mit den zu den ersten Verschlussmitteln des Bauchelementes korrespondierenden zweiten Verschlussmittel das Hinterteil. In einer bevorzugten Ausführungsform umfasst die erfindungsgemäße Stabilisierungsvorrichtung ein Vorderteil. In einer Ausführungsform bildet das Brustelement und das Schulterelement das Vorderteil.

In einer bevorzugten Ausführungsform weist das Vorderteil ein flächig ausgebildetes Brustelement, welches im Bereich der Brust anordbar ausgebildet ist, und ein flächig ausgebildetes Schulterelement, welches im Bereich der Schulter anordbar ausgebildet ist, auf.

In einer Ausführungsform sind Hinterteil und Vorderteil lösbar miteinander verbunden.

In einer Ausführungsform sind Hinterteil und Vorderteil miteinander durch mindestens ein lösbares Verbindungsmittel verbindbar.

Das lösbare Verbindungsmittel verbindet entweder Bauchelement mit Brustelement und/oder Rückenelement mit Schulterelement (ein lösbares Verbindungsmittel zwischen Bauchelement und Schulterelement oder zwischen Rückenelement und Brustelement ist nicht möglich). In einer bevorzugten Ausführungsform weist das Bauchelement ein lösbares Verbindungsmittel zum Brustelement auf. In einer weiteren Ausführungsform weist das Rückenelement ein lösbares Verbindungsmittel zum Schulterelement auf.

In einer weiteren Ausführungsform sind Hinterteil und Vorderteil durch zwei lösbare Verbindungsmittel miteinander verbunden. Dabei weist das Bauchelement ein lösbares Verbindungsmittel zum Brustelement und das Rückenelement ein lösbares Verbindungsmittel zum Schulterelement auf, wobei die lösbaren Verbindungsmittel mit dem Brustelement und dem Schulterelement jeweils lösbar verbunden sind.

In einer Ausführungsform weist das lösbare Verbindungsmittel mindestens ein erstes und mindestens ein zweites Verschlussmittel sowie eine Verbindung auf.

In einer Ausführungsform umfassen die ersten Verschlussmittel des lösbaren Verbindungsmittels zwischen Hinterteil und Vorderteil mechanische Verschlussmittel wie Schnallen, insbesondere Rollschnallen, Doppelsteg-Schnallen, Dreisteg-Schnallen, Leiterschnallen, Schiebeschnallen, Klemmschnallen, Klickschnallen (Blitz- oder Klickverschluss, auch Klickschließen genannt), Steckschnallen, oder Formschlussschnallen. In eher weiteren Ausführungsform umfassen die ersten Verschlussmittel des lösbaren Verbindungsmittels zwischen Hinterteil und Vorderteil mechanische Verschlussmittel wie Knebelverschlüsse, Klettverschlüsse, Druckknöpfe oder Reißverschlüsse. In einer weiteren Ausführungsform umfassen die ersten Verschlussmittel des lösbaren Verbindungsmittels mechanische Verschlussmittel wie Steckschlösser. In einer weiteren Ausführungsform ist das erste Verschlussmittel des lösbaren Verbindungsmittels ein Karabinerhaken mit einem entsprechenden Gegenstück als zweitem Verschlussmittel des lösbaren Verbindungsmittels. In einer weiteren Ausführungsform weisen die ersten Verschlussmittel des lösbaren Verbindungsmittels Verstellschieber auf, wodurch vorteilhaft die Länge der Verbindung zwischen Vorderteil und Hinterteil eingestellt werden kann.

In einer Ausführungsform ist an dem Vorderteil für die Aufnahme des ersten Verschlussmittels des lösbaren Verbindungsmittels des Hinterteils ein korrespondierendes zweites Verschlussmittel angeordnet.

In einer Ausführungsform stellen die zweiten Verschlussmittel des lösbaren Verbindungsmittels die korrespondierenden Gegenstücke zu den ersten Verschlussmitteln des lösbaren Verbindungsmittels dar, sodass vorteilhaft die ersten und zweiten Verschlussmittel formschlüssig und lösbar miteinander verbindbar ausgebildet sind.

In einer Ausführungsform weist das Brustelement oder das Bauchelement ein korrespondierendes zweites Verschlussmittel für die Aufnahme des ersten Verschlussmittels des lösbaren Verbindungsmittels vom Bauchelement oder vom Brustelement auf. In einer weiteren Ausführungsform weist das Rückenelement oder das Schulterelement ein korrespondierendes zweites Verschlussmittel für die Aufnahme des ersten Verschlussmittels des lösbaren Verbindungsmittels vom Schulterelement oder vom Rückenelement auf.

Die zweiten Verschlussmittel des lösbaren Verbindungsmittels sind dabei vorteilhaft so an dem Brustelement oder Bauchelement angeordnet, dass diese mit den ersten Verschlussmitteln des lösbaren Verbindungsmittels des Bauchelementes oder des Brustelementes für den Benutzer einfach zugänglich und zu verbinden sind.

In einer Ausführungsform sind das erste oder das zweite Verschlussmittel des lösbaren Verbindungsmittels punktuell am Bauchelement und/oder am Rückenelement angeordnet. In einer weiteren Ausführungsform sind das erste und das zweite Verschlussmittel des lösbaren Verbindungsmittels punktuell zu der Längsachse des Bauchelementes und/oder des Rückenelementes angeordnet. In einer weiteren Ausführungsform sind das erste oder das zweite Verschlussmittel des lösbaren Verbindungsmittels punktuell an einem Ende der Längsachse des Bauchelementes oder des Rückenelementes angeordnet.

In einer Ausführungsform ist die Verbindung des lösbaren Verbindungsmittels am Hinterteil nicht lösbar angeordnet verbunden, beispielsweise durch Vernähen. In einer weiteren Ausführungsform ist die Verbindung des lösbaren Verbindungsmittels an dem Bauchelement und/oder an dem Rückenelement nicht lösbar angeordnet verbunden, beispielsweise durch Vernähen.

In einer Ausführungsform ist die Verbindung des lösbaren Verbindungsmittels am Vorderteil nicht lösbar angeordnet verbunden, beispielsweise durch Vernähen. In einer weiteren Ausführungsform ist die Verbindung des lösbaren Verbindungsmittels an dem Brustelement und/oder an dem Schulterelement nicht lösbar angeordnet verbunden, beispielsweise durch Vernähen.

In einer Ausführungsform ist die Verbindung des lösbaren Verbindungsmittels als Gurt oder Band ausgestaltet. In einer bevorzugten Ausführungsform ist die Verbindung des lösbaren Verbindungsmittels als Gurtband ausgestaltet. In einer weiteren Ausführungsform weist die Verbindung des lösbaren Verbindungsmittels eine einstellbare Länge und Breite auf.

In einer Ausführungsform umfasst die Stabilisierungsvorrichtung weiterhin eine Tasche.

In einer Ausführungsform nimmt die Tasche die Stabilisierungsvorrichtung zum Transport derselben auf.

In einer Ausführungsform ist die Tasche als Rucksack und/oder als Beutel ausgestaltet.

In einer Ausführungsform weist die Tasche ein Öffnungsmittel, wie beispielsweise einen Reißverschluss, auf. Um die Tasche zu öffnen, wird das Öffnungsmittel geöffnet und die Stabilisierungsvorrichtung kann in die Tasche eingebracht werden. Vorteilhaft lässt sich die Stabilisierungsvorrichtung so transportieren.

In einer bevorzugten Ausführungsform weist die Tasche ein integriertes Trageelement auf. In einer weiteren bevorzugten Ausführungsform ist das Trageelement lösbar von der Tasche ausgebildet und kann an diese bei Bedarf an geeignete erste Aufnahmevorrichtung an der Stabilisierungsvorrichtung befestigt werden. Hierfür weist das Trageelement der Tasche eine zweite Aufnahmeeinrichtung auf. Das Trageelement und die erste Aufnahmevorrichtung der Tasche sind dabei analog zum Trageelement und der ersten Aufnahmevorrichtung der Stabilisierungsvorrichtung ausgestaltet. Vorteilhaft kann die Tasche so benutzerfreundlich transportiert werden. Weiterhin kann das Trageelement der Tasche auch an die Stabilisierungsvorrichtung lösbar befestigt werden.

In einer bevorzugten Ausführungsform ist die Tasche als Decke verwendbar ausgebildet. Dazu wird die Tasche geöffnet, die Stabilisierungsvorrichtung entnommen und die Tasche flächig zur Decke ausgebreitet. Vorteilhaft entspricht die Fläche der ausgebreiteten Tasche in geöffnetem Zustand mindestens der Auflagefläche des Tieres, welches auf der sich so ergebenden Decke Platz nimmt.

In einer Ausführungsform weist die Tasche in ihr nicht lösbare verbundene Verbindungselemente auf. In einer bevorzugten Ausführungsform sind an die mit der Tasche nicht lösbar verbundenen Verbindungselemente lösbare erste Verschlussmittel angeordnet. In einer weiteren bevorzugten Ausführungsform weist die Tasche an ihr befestigte, zu den ersten Verschlussmitteln korrespondierende zweite Verschlussmittel auf.

In einer bevorzugten Ausführungsform ist die Tasche als Umhang wie beispielsweise als Regencape verwendbar ausgebildet. Dazu wird die Tasche geöffnet, die Stabilisierungsvorrichtung entnommen und die geöffnete Tasche flächig auf den Rücken des Tieres gelegt. Die in der Tasche nicht lösbar verbundenen Verbindungselemente werden mit den lösbaren ersten Verschlussmitteln um den Rumpf des Tieres gelegt und befestigt. Vorteilhaft entspricht die Fläche der ausgebreiteten Tasche in geöffnetem Zustand mindestens der Auflagefläche des Rückens des Tieres, auf welchem der sich so ergebende Umhang gelegt wird. Die lösbaren ersten Verschlussmittel der Tasche und die korrespondierenden zweiten Verschlussmittel sind dabei analog zu den lösbaren ersten Verschlussmitteln der Stabilisierungsvorrichtung und den korrespondierenden zweiten Verschlussmitteln ausgestaltet. Vorteilhaft kann das Tier selbst beim Auflegen der geöffneten Tasche als Umhang die erfindungsgemäße Stabilisierungsvorrichtung tragen.

In einer Ausführungsform besteht die Tasche zumindest teilweise aus einem textilen Material. In einer Ausführungsform besteht die Tasche vollständig aus einem textilen Material. Vorteilhaft ist das textile Material robust ausgebildet. In einer Ausführungsform handelt es sich bei dem robusten textilen Material der Tasche um dasselbe robuste textile Material, wie es auch die Stabilisierungsvorrichtung aufweist.

In einer Ausführungsform ist die Tasche zumindest teilweise gepolstert. In einer Ausführungsform ist die Tasche vollständig gepolstert. In einer bevorzugten Ausführungsform ist die Tasche an die Größe der Bestandteile der Stabilisierungsvorrichtung und somit an die Größe des Tieres angepasst. In einer Ausführungsform ist die Tasche an die Größe der Bestandteile und Verbindungselemente der Stabilisierungsvorrichtung und somit an die Größe des Tieres angepasst.

In einer bevorzugten Ausführungsform weist die Tasche zumindest teilweise eine angeordnete Temperiervorrichtung auf.

In einer bevorzugten Ausführungsform wird die erfindungsgemäße Stabilisierungsvorrichtung zur medizinischen Behandlung, zur Entlastung, Stabilisation, Sicherung sowie dem waagerechten Anheben und Transport von Tieren verwendet.

Die erfindungsgemäße Stabilisierungsvorrichtung liegt an anatomisch relevanten und geeigneten Körperstellen des Tieres an. Unter anatomisch relevanten und geeigneten Positionen sind erfindungsgemäß jene Positionen zu verstehen, welche das Tier in seiner Bewegung nicht hindern oder schmerzen.

In einer Ausführungsform sind die Bestandteile der erfindungsgemäßen Stabilisierungsvorrichtung individuell in variablen und unterschiedlichen Abmessungen gestaltbar. Dadurch kann man die Bestandteile auch in der Größe an die entsprechenden Körperteile auswählen und anpassen.

Der Bewegungsapparat des Tieres soll entlastet werden. Dazu zählen insbesondere die Gliedmaßen, Gelenke, Sehnen, Muskeln und die Wirbelsäule. Die Wirbelsäule wird beim Anheben des Tieres nicht verdreht, sondern seitlich arretiert und stabilisiert, wodurch unerwünschte Zerrungen sowie unerwünschte Verdrehungen des Rumpfes des Tieres verhindert werden.

Vorteilhaft werden durch die erfindungsgemäße Stabilisierungsvorrichtung keine Muskeln oder Blutgefäße eingeklemmt. Dadurch wird die Ausübung von Druck auf Körperbereiche oder punktuelle Stellen des Tieres verhindert, was zum Wohlbefinden beim Tragen der Stabilisierungsvorrichtung beiträgt.

Für das Tier selbst wird der Tragekomfort gesteigert. Weiterhin vorteilhaft wird durch die Ausgestaltung der Auflagefläche des Bauchelementes beispielweise eine muskel-, gelenk- und blutgefäßschonende Behandlung des Tieres gewährleistet. Somit wird der Bewegungsapparat des Tieres geschont.

Weiterhin ermöglicht die erfindungsgemäße Stabilisierungsvorrichtung eine körpergerechte Haltung für den Benutzer oder Halter des Tieres. Der Halter kann beim Anheben des Tieres oder beim Gassi gehen durch die Ausgestaltung der Stabilisierungsvorrichtung eine rückenschonende Haltung und einen aufrechten Gang einnehmen.

Zudem dient die erfindungsgemäße Stabilisierungsvorrichtung der ganzheitlichen Unterstützung des Tieres im Alter. Dies betrifft insbesondere Tiere, welche altersmäßig nicht mehr in der Lage sind, lange Strecken zu laufen.

Auch nach Verletzungen oder Erkrankungen der Muskulatur, der Gliedmaßen und Gelenke ist die erfindungsgemäße Stabilisierungsvorrichtung verwendbar ausgebildet. Dabei kann das Tier mit der erfindungsgemäßen Stabilisierungsvorrichtung fixiert und stabil gelagert werden. Dies trifft auch auf eine ruhende Position zu. Die erfindungsgemäße Stabilisierungsvorrichtung kann durch die Möglichkeit, es in Einzelteile zu zerlegen, auch einfach und bequem an ein liegendes Tier angebracht werden, ohne dass sich dieser bewegen oder durch eine Öffnung steigen muss. Dies kann verletzungsbedingt sein oder auch, wenn das Tier unter Narkose steht und trotzdem bewegt oder transportiert werden muss. Die erfindungsgemäße Stabilisierungsvorrichtung ist somit für den Benutzer einfach handhabbar, ohne das Tier unnötig zu belasten.

Der medizinische Einsatz der erfindungsgemäßen Stabilisierungsvorrichtung begründet sich weiterhin in der Verwendung nach Operationen, von welcher sich die Tiere erholen müssen. Häufig kommt es bei den Tieren infolge einer Operation zu einer Schwächung und Veränderung der Anatomie, beispielsweise durch Muskelschwund infolge des Abnehmens nach einer Operation. Vorteilhaft kann hier auf die passgenaue und anatomisch schonende Stabilisierungsvorrichtung zurückgegriffen werden, welche individuell an die jeweilig vorliegenden anatomischen Proportionen anpassbar ist und muskelschonend als Gehhilfe anlegbar ist.

Durch die erfindungsgemäße Stabilisierungsvorrichtung kann das Tier dadurch nahezu waagerecht angehoben und transportiert werden. Beispielsweise kann somit kurz- oder längerfristige Hilfe beim Treppensteigen oder beim Überwinden von Hindernissen wie Türschwellen gewährleistet werden. Im Sinne der Erfindung bedeutet eine waagerechte Ausrichtung des Tieres dabei die zur Schwerkraftrichtung senkrechte Ausrichtung des Rumpfes. Der Rumpf des Tieres ist somit parallel zum Boden ausgerichtet, was der natürlichen Haltung des Tieres im Stand entspricht. Somit wird dem Bedürfnis des Tieres entsprochen, wonach dieser bestrebt ist, stets mit allen vier Beinen auf dem Boden aufzukommen.

Benutzer der erfindungsgemäßen Stabilisierungsvorrichtung sind beispielsweise Hundehalter, welche die Stabilisierungsvorrichtung zum Gassi gehen verwenden möchten oder die ihren Hund nach Operationen oder altersbedingt anheben und tragen müssen.

Weiterhin kann die erfindungsgemäße Stabilisierungsvorrichtung im Outdooreinsatz verwendet werden. Zudem kann die erfindungsgemäße Stabilisierungsvorrichtung auch von Rettungsinstitutionen, wie beispielsweise der Bergwacht, der Polizei, der Feuerwehr, Rettungskräften oder sonstigen Bergungskräften beispielsweise in einem unwegsamen Gelände wie beispielsweise im Hochgebirge als Zug-, Such und Rettungsgeschirr für Rettungshunde verwendet werden. Auch für im Militär eingesetzte Tiere oder Tiere zur Jagd wie beispielsweise Jagdhunde, kann die erfindungsgemäße Stabilisierungsvorrichtung eingesetzt werden. Zudem kann die erfindungsgemäße Stabilisierungsvorrichtung auch von Extremsportlern wie Kletterern in einem unwegsamen Gelände wie beispielsweise im Hochgebirge verwendet werden. Dabei kann das Tier, beispielsweise ein Hund, körperschonend abgeseilt werden, indem er in einer nahezu waagerechten Position bleibt. Das Tier bleibt dabei ruhig, da diese nahezu waagerechte Position auch seiner natürlichen Standposition (alle vier Beine weisen zum Boden) entspricht. Zudem wird das Tier durch die Stabilisierungsvorrichtung stabilisiert. Weiterhin kann die erfindungsgemäße Stabilisierungsvorrichtung einfach und schnell angelegt und wieder abgenommen werden.

Weiterhin sind als Benutzer der erfindungsgemäßen Stabilisierungsvorrichtung sämtliche Halter von Tieren vorgesehen.

Außerdem sind als Benutzer der erfindungsgemäßen Stabilisierungsvorrichtung Tierärzte, Tier-Rehakliniken oder Tier-Therapieschulen geeignet, welche die frisch operierten Tiere überwachen und diese beispielsweise in Wasserbädern wieder trainieren. Dabei kann die erfindungsgemäße Stabilisierungsvorrichtung zur Stabilisierung und zur Entlastung von Teilmuskulatur selbst im Wasser getragen werden.

Für die Realisierung der Erfindung ist es auch zweckmäßig, die vorbeschriebenen erfindungsgemäßen Ausgestaltungen, Ausführungsformen und Merkmale der Ansprüche in jeder Anordnung miteinander zu kombinieren.

### Ausführungsbeispiele

Nachfolgend soll die Erfindung anhand eines Ausführungsbeispieles eingehender erläutert werden. Das Ausführungsbeispiel bezieht sich auf eine Stabilisierungsvorrichtung für Hunde und soll dabei die Erfindung beschreiben ohne diese zu beschränken.

Die Stabilisierungsvorrichtung für Hunde umfasst ein Bauchelement, ein Rückenelement, ein Brustelement und ein Schulterelement. Die einzelnen Elemente sind über Gurtbänder miteinander verbunden.

Anhand von Zeichnungen wird die Erfindung näher erläutert. Dabei zeigen
- **Fig. 1**: Bauchelement der Stabilisierungsvorrichtung für Hunde
- **Fig. 2**: Rückenelement der Stabilisierungsvorrichtung für Hunde
- **Fig. 3**: Brustelement der Stabilisierungsvorrichtung für Hunde
- **Fig. 4**: Schulterelement der Stabilisierungsvorrichtung für Hunde

Die in den Figuren dargestellten oberen Teile der Bestandteile zeigen jeweils in Richtung Kopf des Hundes, die in den Figuren dargestellten unteren Teile der Bestandteile zeigen in Richtung Rute des Hundes. Die jeweils von oben nach unten eingezeichneten Längsachsen der Bestandteile verlaufen entlang der Wirbelsäule des Hundes und beziehen sich auf dessen Laufrichtung.

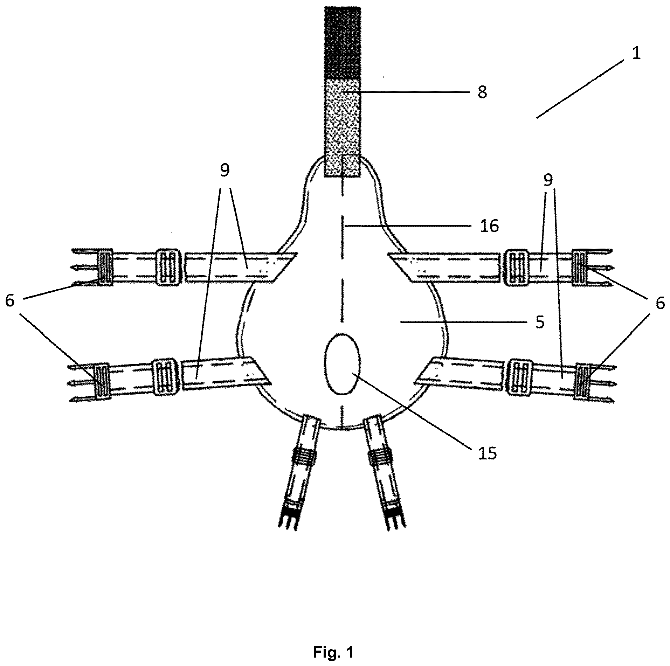

**Figur 1** zeigt ein Bauchelement 1, welches eine birnenförmige Auflagefläche 5 aufweist.

Das Bauchelement 1 weist vier punktuell daran angeordnete Gurtbänder 9 auf, welche spiegelsymmetrisch zu der Längsachse 16 des Bauchelementes 1 und voneinander beabstandet angeordnet sind. Dabei sind zwei Gurtbänder 9 auf der linken Seite und zwei Gurtbänder 9 auf der rechten Seite der Auflagefläche 5 des Bauchelementes 1 relativ zur Längsachse 16 angeordnet. Zusätzlich zu den vier punktuell angeordneten Gurtbändern 9 sind zwei schmaler ausgeführte Gurtbänder 9 an der in der Figur unteren Seite des Bauchelementes 1 dargestellt, durch welche die Rute des Hundes geführt wird. Die Gurtbänder 9 sind alle in der Länge durch Verstellschieber verstellbar.

Zentral und symmetrisch um die Längsachse 16 im Bauchelement 1 angeordnet ist die Aussparung für die Durchführung des Geschlechtsorgans eines Rüden 15 vorgesehen.

Die Gurtbänder 9 sind mit dem Bauchelement vernäht. An jedem Gurtband 9 ist eine Klickschnalle 6 angeordnet, über welche das Bauchelement 1 mit dem Rückenelement 2 verbunden wird (Verbindung und Klickschnallen in der Figur nicht gezeigt).

Das Bauchelement 1 weist oben einen parallel zur Längsachse 16 verlaufenden Verbindungsgurt 8 als lösbares Verbindungsmittel auf, durch welche das Bauchelement 1 mit einem Brustelement 3 verbunden wird (Brustelement und Verbindung in der Figur nicht gezeigt). Der Verbindungsgurt 8 ist, ebenfalls wie die Gurtbänder 9, mit dem Bauchelement 1 vernäht.

Das Bauchelement 1 ist gepolstert und weist ein Abstandsgewirke auf (in der Figur nicht dargestellt).

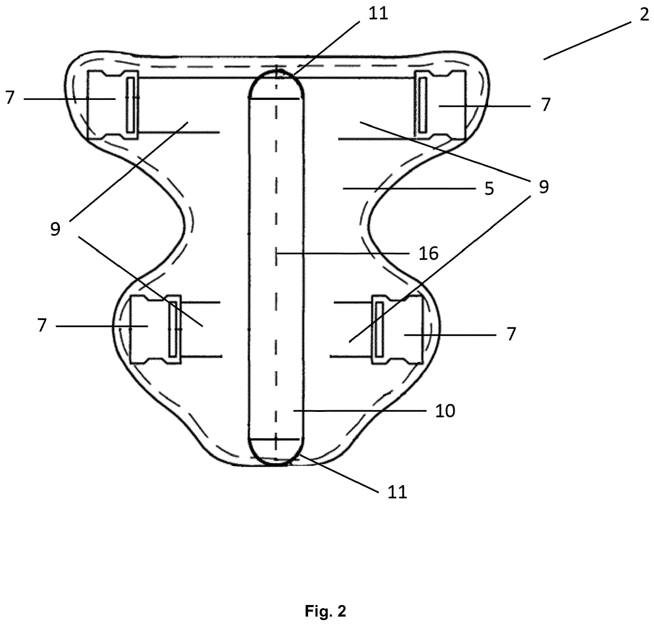

**Figur 2** zeigt ein Rückenelement 2, welches eine flächig ausgebildete Auflagefläche 5 aufweist. Das Rückenelement 2 weist vier Gurtbänder 9 auf, welche parallel senkrecht und voneinander beabstandet zur Längsachse 16 an das Rückenelement 2 angenäht sind und spiegelsymmetrisch zu der Längsachse 16 angeordnet sind. An jedem Gurtband 9 ist jeweils ein Gegenstück 7 zu der am Bauchelement 1 angebrachten Klickschnalle 6 angeordnet, über welche das Bauchelement 1 mit dem Rückenelement 2 lösbar verbunden wird (Verbindung in der Figur nicht gezeigt).

Am Rückenelement 2 ist eine Rückenschiene 10 aus Kunststoff befestigt, welche unter den über die Rückenschiene 10 des Rückenelementes 2 verlaufenden Gurtbändern 9 vernäht ist. Die Rückenschiene 10 verläuft durchgehend entlang der Längsachse 16 des Rückenelementes 2 und somit entlang der Wirbelsäule bis zum Schwanzansatz des Hundes.

Weiterhin weist das Rückenelement 2 an seinem oberen und unteren Ende entlang der Längsachse 16 jeweils einen D-Ring 11 auf. Dabei sind die D-Ringe 11 an zwei voneinander entlang der Längsachse 16 meist beabstandetsten Stellen des Rückelementes 2 mit einem Gurtband 9 vernäht. In die D-Ringe 11 kann jeweils das Ende eines Tragegriffs eingehakt werden (in der Figur nicht gezeigt).

Das Rückenelement 2 ist gepolstert (in der Figur nicht gezeigt).

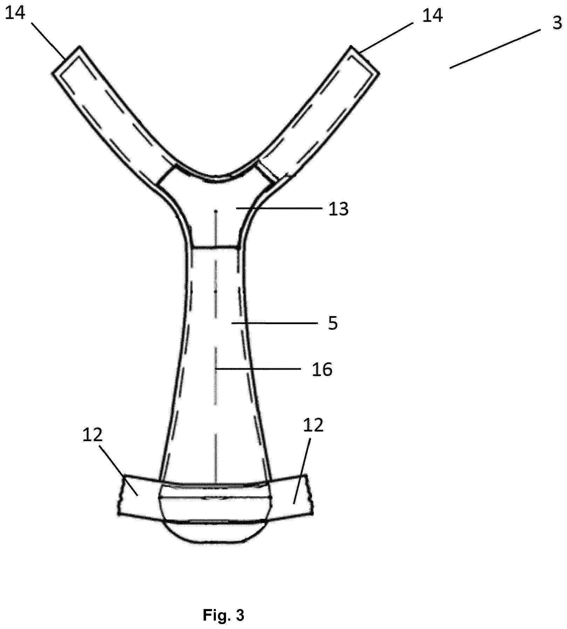

**Figur 3** zeigt ein Brustelement 3, welches eine stimmgabelförmige Auflagefläche 5 aufweist.

Das Brustelement 3 weist unten zwei punktuell am Brustelement 3 angeordnete Gurtbänder 12 auf, welche jeweils spiegelsymmetrisch zu der Längsachse 16 des Brustelementes 3 angeordnet sind. Dabei verlaufen die Gurtbänder 12 durchgehend über das Brustelement 3. An den Gurtbändern 12 ist jeweils eine daran angeordnete Klickschnalle angebracht (in der Figur nicht gezeigt).

Das Brustelement 3 weist an den Enden der beiden Zinken der stimmgabelförmigen Auflagefläche 5 jeweils eine lösbare Verbindung 14 auf, durch welche das Brustelement 3 mit dem Schulterelement 4 lösbar verbunden wird (Schulterelement in der Figur nicht gezeigt).

Das Brustelement 3 weist unten einen Klettverschluss für den vom Bauchelement 1 kommenden Verbindungsgurt 8 auf, durch welche das Brustelement 3 mit einem Bauchelement 1 verbunden wird (Verbindungsgurt und Klettverschluss in der Figur nicht gezeigt). Der Verbindungsgurt 8 ist dabei ebenfalls wie die Gurtbänder (9 und 12) mit dem Bauchelement 1 vernäht.

Das Brustelement 3 weist eine Brustbeinpolsterung 13 und ein Abstandsgewirke auf (Abstandsgewirke in der Figur nicht gezeigt).

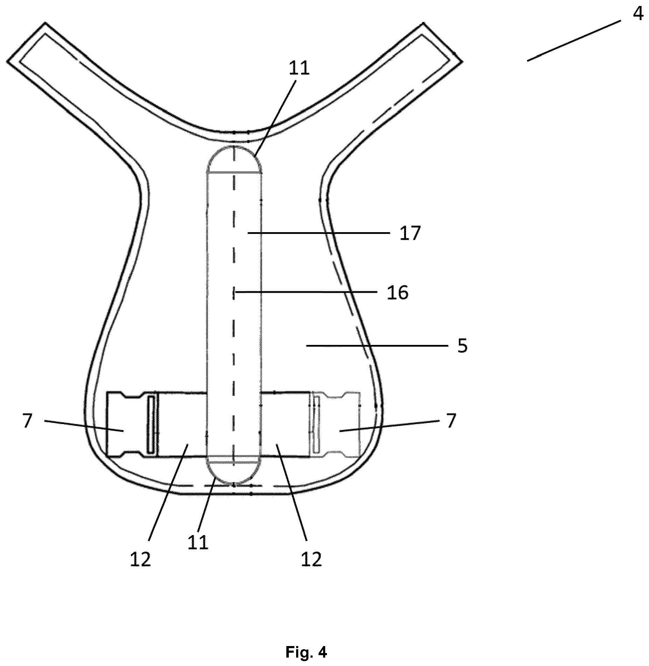

**Figur 4** zeigt ein Schulterelement 4, welches eine stimmgabelförmige Auflagefläche 5 aufweist, die breiter als beim Brustelement 3 ausgestaltet ist.

Das Schulterelement 4 weist unten zwei punktuell am Schulterelement 4 angeordnete Gurtbänder 12 auf, welche jeweils spiegelsymmetrisch zu der Längsachse 16 des Schulterelementes 4 angeordnet sind. Dabei verlaufen die Gurtbänder 12 durchgehend über das Schulterelement 4. An jedem Gurtband 12 ist jeweils ein Gegenstück 7 zu der am Brustelement 3 angebrachten Klickschnalle 6 angeordnet, über welche das Brustelement 3 mit dem Schulterelement 4 lösbar verbunden wird (Brustelement und Verbindung in der Figur nicht gezeigt).

Das Schulterelement weist weiterhin an den Enden seiner beiden Zinken einen Klickverschluss auf, durch welche das Schulterelement mit dem Brustelement lösbar verbunden wird (in der Figur nicht gezeigt).

Am Schulterelement 4 ist eine Schulterschiene 17 aus Kunststoff befestigt, welche unter den über die Schulterschiene 17 des Schulterelementes 4 verlaufenden Gurtbändern 12 vernäht ist. Die Schulterschiene 17 verläuft durchgehend entlang der Längsachse 16 des Schulterelementes 4 und somit entlang der Wirbelsäule des Hundes.

Weiterhin weist das Schulterelement 4 an seinem oberen und unteren Ende entlang der Längsachse 16 jeweils einen D-Ring 11 auf. Dabei sind die D-Ringe 11 an zwei voneinander entlang der Längsachse 16 meist beabstandetsten Stellen des Schulterelementes 4 mit einem Gurtband 12 vernäht. In die D-Ringe 11 kann jeweils das Ende eines Tragegriffs eingehakt werden (in der Figur nicht gezeigt). Die beiden Enden eines Tragegriffes können zudem auch in einen D-Ring 11 am Schulterelement 4 und in einen D-Ring 11 am Rückenelement 2 eingehakt werden, wodurch der Hund sowohl mittels des Schulterelementes 4 und des Rückenelementes 2 getragen werden kann.

Das Schulterelement 4 ist gepolstert (in der Figur nicht gezeigt).

### Bezugszeichen

- 1: Bauchelement
- 2: Rückenelement
- 3: Brustelement
- 4: Schulterelement
- 5: Auflagefläche
- 6: Klickschnalle
- 7: Gegenstück zur Klickschnalle
- 8: Lösbarer Verbindungsgurt (am Bauchelement) zum Brustelement
- 9: Lösbares Gurtband (am Bauchelement) zum Rückenelement
- 10: Rückenschiene
- 11: D-Ringe zur Befestigung von Tragegriffen
- 12: Lösbares Gurtband (am Brustelement) zum Schulterelement
- 13: Brustbeinpolsterung
- 14: Lösbare Verbindung (am Brustelement) zum Schulterelement
- 15: Aussparung für die Geschlechtsorgane von Rüden
- 16: Längsachse
- 17: Schulterschiene

## Patentansprüche

1. Stabilisierungsvorrichtung für Tiere zur medizinischen Behandlung, zur Entlastung, Stabilisation, Sicherung sowie dem waagerechten Anheben und Transport, umfassend mindestens folgende Bestandteile:
ein im Wesentlichen flächig ausgebildetes Bauchelement, welches
- im Bereich des Bauches anordbar ausgebildet ist, aufweisend
a) eine flexible und anatomisch anpassbare Auflagefläche und
b) mindestens vier Verbindungselemente mit daran angeordneten ersten Verschlussmitteln,
sowie ein flächig ausgebildetes Rückenelement, welches
- im Bereich des Rückens anordbar ausgebildet ist, aufweisend
c) zumindest abschnittsweise zu der Längsachse mindestens ein Mittel zur Stabilisierung und
d) zumindest vier zweite Verschlussmittel aufweist,
wobei
- die mindestens vier Verbindungselemente spiegelsymmetrisch zu der Längsachse des Bauchelementes voneinander beabstandet am Bauchelement angeordnet sind,
- das Bauchelement mit dem Rückenelement durch die Verbindungselemente mit den daran angeordneten ersten Verschlussmitteln und den korrespondierenden zweiten Verschlussmitteln lösbar verbindbar ausgebildet ist, wobei die Anzahl der ersten Verschlussmittel der Anzahl der korrespondierenden zweiten Verschlussmittel entspricht.

2. Stabilisierungsvorrichtung für Tiere nach Anspruch 1, **dadurch gekennzeichnet, dass** zumindest ein Teil der Bestandteile der Stabilisierungsvorrichtung ein unelastisches Abstandsgewirke mit einer Dehnfähigkeit zwischen 0% und 15% aufweist.

3. Stabilisierungsvorrichtung für Tiere nach Anspruch 1 oder 2, weiterhin umfassend ein Vorderteil, welches ein flächig ausgebildetes Brustelement, welches im Bereich der Brust anordbar ausgebildet ist, und ein flächig ausgebildetes Schulterelement, welches im Bereich der Schulter anordbar ausgebildet ist, aufweist.

4. Stabilisierungsvorrichtung für Tiere nach einem der Ansprüche 1 bis 3, **dadurch gekennzeichnet, dass** das Brustelement mit dem Schulterelement durch mindestens zwei Verbindungselemente verbunden ist.

5. Stabilisierungsvorrichtung für Tiere nach einem der Ansprüche 1 bis 4, **dadurch gekennzeichnet, dass** das Bauchelement ein lösbares Verbindungsmittel zum Brustelement aufweist.

6. Stabilisierungsvorrichtung für Tiere nach einem der Ansprüche 1 bis 5, **dadurch gekennzeichnet, dass** diese mindestens zwei erste Aufnahmeeinrichtungen aufweist, wobei die ersten Aufnahmeeinrichtungen voneinander beabstandet am Rückenelement und/oder am Schulterelement angeordnet sind.

7. Stabilisierungsvorrichtung für Tiere nach einem der Ansprüche 1 bis 6, **dadurch gekennzeichnet, dass** in die ersten Aufnahmeeinrichtungen mindestens ein lösbares Trageelement, welches mindestens zwei zweite Aufnahmeeinrichtungen aufweist, anbringbar ist.

8. Stabilisierungsvorrichtung für Tiere nach einem der Ansprüche 1 bis 7, **dadurch gekennzeichnet, dass** in mindestens einem Bestandteil zumindest teilweise eine Temperiervorrichtung angebracht ist.

9. Stabilisierungsvorrichtung für Tiere nach einem der Ansprüche 1 bis 8, **dadurch gekennzeichnet, dass** das Bauchelement eine Aussparung für das Geschlechtsorgan bei männlichen Tieren aufweist.

10. Stabilisierungsvorrichtung für Tiere nach einem der Ansprüche 1 bis 9, weiterhin umfassend eine Vorrichtung zur Erfassung von Vitalfunktionen von Tieren und/oder zur Ortung und/oder Überwachung und/oder Geodatenübermittlung von Tieren.

11. Tasche zur Aufnahme einer Stabilisierungsvorrichtung für Tiere nach einem der Ansprüche 1 bis 10, **dadurch gekennzeichnet, dass** die Tasche ein integriertes lösbares Trageelement sowie Verbindungselemente mit daran angeordneten ersten Verschlussmitteln sowie korrespondierende zweite Verschlussmittel aufweist und als Decke oder Umhang mit zumindest teilweise angebrachter Temperiervorrichtung verwendbar ausgebildet ist.

12. Verwendung einer Stabilisierungsvorrichtung für Tiere nach einem der Ansprüche 1 bis 10 zur medizinischen Behandlung, zur Entlastung, Stabilisation, Sicherung sowie dem waagerechten Anheben und Transport von Tieren sowie einer Tasche nach Anspruch 11 zur Aufnahme einer Stabilisierungsvorrichtung nach einem der Ansprüche 1 bis 10.
